# EUROPEAN PATENT APPLICATION

(11) **EP 4 119 669 A1**
(43) Date of publication of application: **18.01.2023**
(21) Application number: 21768052.9
(22) Date of filing: 12.03.2021
(51) Int. Cl.: C12P 7/44, C12N 1/21

(54) **METHOD FOR PRODUCING 2-PYRONE-4,6-DICARBOXYLIC ACID**

(30) Priority: 13.03.2020 JP 2020044525
(71) Applicant: Forest Research and Management Organization, Tsukuba-shi Ibaraki 305-8687 (JP); Kantechs Co. Ltd, Tokyo 112-0004 (JP); National University Corporation Nagaoka University of Technology, Nagaoka-shi Niigata 940-2188 (JP)
(72) Inventor: NAKAMURA, Masaya, Tsukuba-shi, Ibaraki 305-8687 (JP); OTSUKA, Yuichiro, Tsukuba-shi, Ibaraki 305-8687 (JP); KAMEYAMA, Toshiji, Tokyo 112-0004 (JP); KAMEYAMA, Koki, Tokyo 112-0004 (JP); MASAI, Eiji, Nagaoka-shi, Niigata 940-2188 (JP); KAMIMURA, Naofumi, Nagaoka-shi, Niigata 940-2188 (JP); KATAYAMA, Yoshihiro, Tokyo 176-0021 (JP)
(74) Representative: AWA Sweden AB
(86) International application number: PCT/JP2021/010235
(87) International publication number: WO 2021/182639

(57) **Abstract**

Provided is a method for producing 2-pyrone-4,6-dicarboxylic acid (PDC) by culturing a microorganism that produces PDC. The present invention provides a method of producing PDC by culturing a microorganism that produces 2-pyrone-4,6-dicarboxylic acid (PDC), wherein the method comprises: dissolving the starting substance for production of PDC in a buffer solution that contains no alkali metals, and adjusting the pH of a culture solution with a buffer solution that contains no alkali metals.

## Description

### FIELD

The present invention relates to a method for producing 2-pyrone-4,6-dicarboxylic acid (PDC) by culturing microorganisms that produce PDC.

### BACKGROUND

Lignin, a major component of plants, is an aromatic polymer compound that is widely distributed throughout plant cell walls and accounts for 30% of the biomass resource for trees and 15% for rice or corn stalks. Plants also contain a wide variety of other aromatic compounds as constituent components. However, effective techniques for plant-derived aromatic components composed mainly of lignin have not been developed because of the varied components present in their chemical structures, and because of their complex macromolecular structures. The methods of use that have been implemented in the prior art involve separation and production of vanillin as a material for perfumes from low molecular aromatic decomposition products generated by chemical decomposition, such as alkaline decomposition of plant-derived aromatic components composed mainly of lignin. It is currently the case, however, that no method exists for effectively using large amounts of low molecular aromatic substances, other than vanillin, that are produced by chemical decomposition. Lignin produced in mass by paper-making processes has therefore been combusted as a substitute for heavy oil, without being effectively utilized.

Development of the modern petrochemical industry has been based on the principle of first carrying out catalytic decomposition and fractional distillation of crude oil, which is a mixture of various chemical components, for conversion to simple intermediates such as benzene and ethylene, and using these as materials to produce different types of functional plastics. The basic concept underlying development of the petrochemical industry is a universal principle which is also applicable to technology that employs plant aromatic components such as lignins having varied chemical structures and complex macromolecular structures.

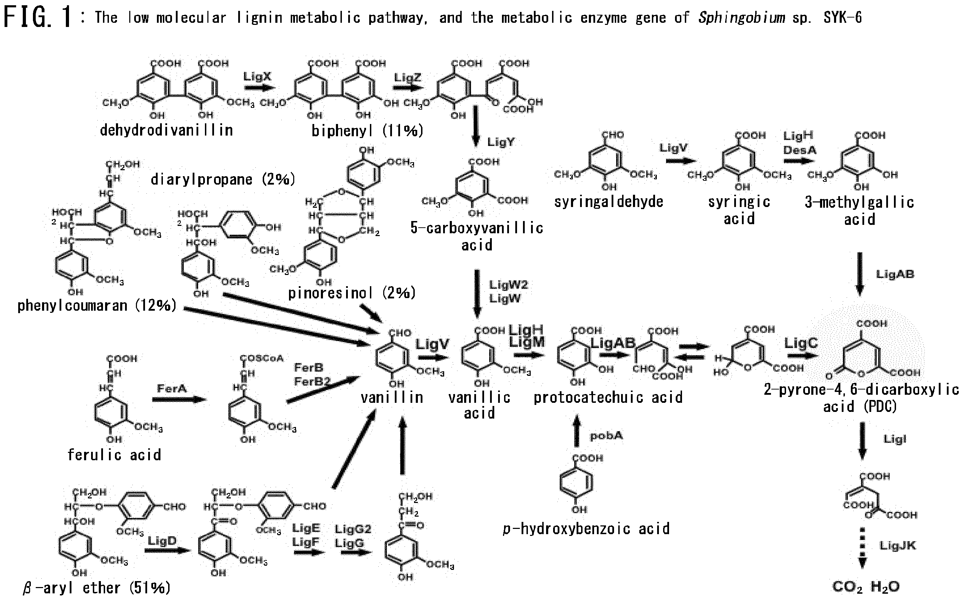

It has been found in the past that plant aromatic components such as lignin are converted to low molecular mixtures containing vanillin, syringaldehyde, vanillic acid, syringic acid, protocatechuic acid and the like by chemical decomposition methods such as hydrolysis, oxidative decomposition or solvent decomposition, or physicochemical decomposition methods with supercritical water or supercritical organic solvents, and it has also been demonstrated that these five compounds are completely metabolized to carbon dioxide and water by the microorganism *Sphingobium* sp. SYK-6, with decomposition of the five compounds in this metabolic pathway being via the single intermediate 2-pyrone-4,6-dicarboxylic acid (see Fig. 1).

The compound 2-pyrone-4,6-dicarboxylic acid (PDC) has two carboxyl groups in the molecule similar to terephthalic acid (TPA) which is a starting material for polyethylene terephthalate (PET) resins, and it is a typical polycondensation material with promising novel properties that currently do not exist in the petrochemical industry.

Methods for producing PDC have been reported, using transformants having genes coding for four enzymes (benzaldehyde dehydrogenase, demethylase, protocatechuate 4,5-dioxygenase and 4-carboxy-2-hydroxymuconate 6-semialdehyde dehydrogenase) that catalyze a multistage reaction process for fermentative production of PDC, to produce PDC from the aforementioned five different compounds (PTL 1 and NPL 1, for example).

It has been disclosed that the method described in PTL 1 yields 10 to 20 g of PDC per 1 L of fermentate, while the method described in NPL 1 produces 15 g/L of PDC. With such yields, however, even production of high-performance polymer materials has been impractical because of the high cost of the final products and their low potential as competitive alternatives in the industry.

PTL 2 discloses a method for purifying PDC which is designed to increase purification efficiency by forming a salt of a metal ion (such as sodium ion) with a PDC product.

PTL 3 discloses a method of producing a recombinant vector for PDC production, and transformants incorporating it.

### [CITATION LIST]

### [PATENT LITERATURE]

[PTL 1] Japanese Unexamined Patent Publication No. 2005-278549
[PTL 2] Japanese Unexamined Patent Publication No. 2008-079603
[PTL 3] Japanese Unexamined Patent Publication No. 2011-067139

### [NON PATENT LITERATURE]

[NPL 1] Otsuka, et al., Journal of the Japan Society for Environmental Biotechnology, (2006), 6(2): 93-103

### SUMMARY

### [TECHNICAL PROBLEM]

In PTL 1 and NPL 1, where sodium hydroxide is used for dissolution and pH adjustment of the starting substances and a sodium salt is also added into the culture medium, these sodium ions form salts with the PDC product, thus insolubilizing it, and with this in mind the present inventors have conducted diligent research on whether PDC production efficiency can be increased by avoiding insolubilization of PDC caused by such PDC salt formation.

It is therefore an object of the present invention to establish a high density culture method for microorganisms that fermentatively produce PDC, in which formation of salts of PDC with metal ions is prevented, and to thus provide a method of producing PDC at high yield. The present invention also examines culture medium components for production of PDC at even higher yield.

### [SOLUTION TO PROBLEM]

As a result of much research in light of the current situation, the present inventors have found that it is possible to prevent formation of salts by using a buffer solution containing no alkali metals as the buffer solution for dissolution of the starting substances and the buffer solution for pH adjustment during production of PDC, and to thus achieve high density culturing of microorganisms in order to produce 2-pyrone-4,6-dicarboxylic acid at high yield, and the present invention has been completed on the basis of this finding.

The present inventors additionally succeeded in preventing salt formation by also using a main culture medium containing no alkali metals, as the culturing medium for the microorganisms.

Specifically:
(1) the invention provides a method of producing 2-pyrone-4,6-dicarboxylic acid (PDC) by culturing a microorganism that PDC, wherein the method comprises:
   dissolving the starting substance for production of PDC in a buffer solution that contains no alkali metals; and
   adjusting pH of a culture solution with a buffer solution that contains no alkali metals.
(2) The invention further provides the method according to (1), wherein:
   the dissolution of the starting substance for production of PDC in the buffer solution that contains no alkali metals comprises culturing the microorganism in the culture solution containing the buffer solution that contains no alkali metals, in which the starting substance has been dissolved; and
   the pH adjustment of the culture solution with the buffer solution that contains no alkali metals comprises appropriate adjustment of the pH of the culture solution with the buffer solution that contains no alkali metals when the pH has been decreased during culturing.
(3) The invention further provides the method according to (1) or (2), wherein the buffer solution that contains no alkali metals is an ammonium-based buffer solution.
(4) The present invention further provides the method according to (3), wherein the ammonium-based buffer solution is ammonia water.
(5) The invention further provides the method according to any one of (1) to (4), which further comprises adding an amino acid mixture to the culture solution.
(6) The invention further provides the method according to (5), wherein yeast extract is used as the amino acid mixture.
(7) The invention further provides the method according to (6), wherein the yeast extract is added to the culture solution in an amount of 3 g/L or more.
(8) The invention provides the method according to any one of (1) to (7), which further comprises adding a carbon source to the culture solution.
(9) The invention further provides the method according to (8), wherein the carbon source is glucose.
(10) The invention further provides the method according to (8) or (9), wherein the carbon source is added by fed-batch addition.
(11) The invention further provides the method according to any one of (1) to (10), which further comprises adding a divalent metal ion to the culture solution.
(12) The invention further provides the method according to (11), wherein the divalent metal ion is composed of MgSO₄·7H₂O, FeSO₄·7H₂O, MgO, CaCO₃, ZnSO₄·7H₂O, MnSO₄·4H₂O, CuSO₄·5H₂O, CoSO₄·7H₂O and H₃BO₃.
(13) The invention further provides the method according to any one of (1) to (12), which further comprises adding the starting substance to the culture solution by fed-batch addition.
(14) The invention further provides the method according to any one of (1) to (13), wherein the microorganism is a transformant of a *Pseudomonas* bacterium that has been transfected with a recombinant vector.
(15) The invention further provides the method according to (14), wherein the *Pseudomonas* bacterium is *Pseudomonas putida.*
(16) The invention further provides the method according to any one of (1) to (15), wherein the starting substance is vanillic acid or vanillin, or a mixture of aromatic substances comprising vanillin and/or vanillic acid.
(17) The invention further provides the method according to any one of (1) to (16), wherein PDC is produced at 40 g or more per culture solution (L).
(18) The invention further provides the method according to (17), wherein PDC is produced at 80 g or more per culture solution (L).
(19) The invention further provides the method according to (18), wherein PDC is produced at 100 g or more per culture solution (L).

### [ADVANTAGEOUS EFFECTS OF INVENTION]

According to the invention it is possible to culture a PDC-producing microorganism at high density, by preventing salt formation between PDC and alkali metal ions, whereby to produce PDC inexpensively and at high yield.

### BRIEF DESCRIPTION OF DRAWINGS

Fig. 1 is a diagram showing the low molecular lignin metabolic pathway, and the metabolic enzyme gene of *Sphingobium* sp. SYK-6.
Fig. 2 is a diagram showing time-dependent change in different values for PDC production from vanillic acid (1).
Fig. 3 is a diagram showing TLC results for PDC production from vanillic acid (1).
Fig. 4 is a diagram showing time-dependent change in different values for PDC production from vanillic acid (2).
Fig. 5 is a diagram showing TLC results for PDC production from vanillic acid (2).

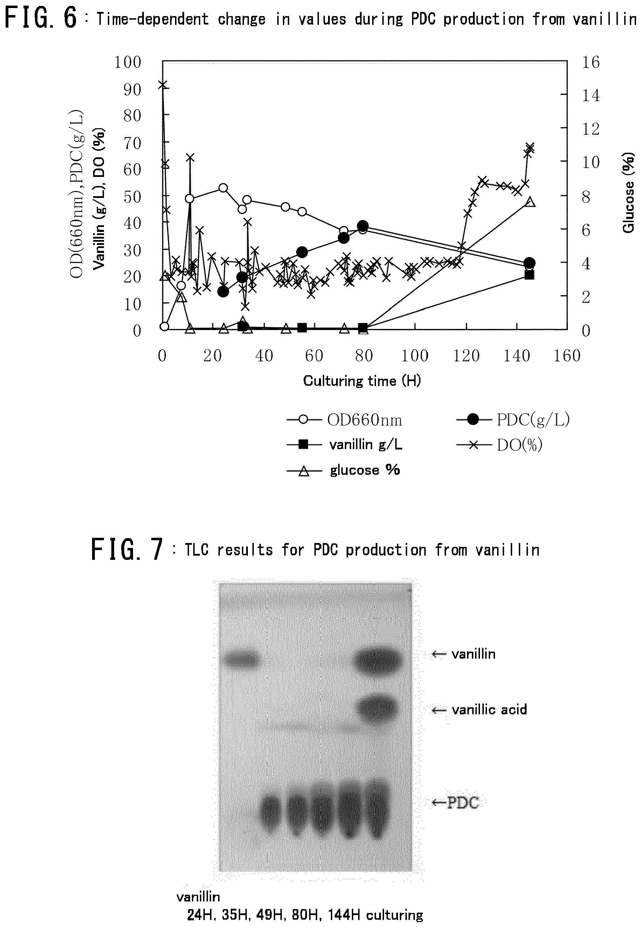

Fig. 6 is a diagram showing time-dependent change in different values for PDC production from vanillin.
Fig. 7 is a diagram showing TLC results for PDC production from vanillin.

### DESCRIPTION OF EMBODIMENTS

The present invention provides a method of producing 2-pyrone-4,6-dicarboxylic acid (PDC)by culturing a microorganism that produces PDC, wherein the method comprises: dissolving the starting substance for production of PDC in a buffer solution that contains no alkali metals; and adjusting the pH of a culture solution with a buffer solution that contains no alkali metals.

By using a buffer solution that contains no alkali metals for culturing, the method of the invention can prevent formation of salts of PDC to allow high density culturing of microorganisms (high-yield PDC production).

The starting substance used for the invention may be vanillic acid or vanillin, for example, as described below. The starting substance is preferably dissolved in a buffer solution, and added to the culture solution in the form of a solution. In results of previous research by the present inventors, residue of vanillin and accumulation of vanillic acid have been seen when vanillin was added in powder form to the culture solution, with virtually no production of PDC. Since a rapid increase in dissolved oxygen was observed immediately after addition of powdered vanillin, it is assumed that addition of vanillin in powder form has an adverse effect on microbial growth.

The pH of the culture solution should be adjusted to a range allowing suitable growth of microorganisms, such as the range of pH 4.0 to 10.0. Since PDC has two carboxyl groups, the pH of the culture solution is decreased as PDC is produced. In order to prevent inhibiting growth of microorganisms due to the lower pH and maintain a suitable pH, it is necessary to appropriately adjust the pH of the culture solution with a buffer solution. The pH of the culture solution is preferably controlled to pH 6.0 or higher, and more preferably to the range of pH 6.5 to 7.5. The pH may be controlled, for example, by automatic addition of buffer solution in response to pH fluctuations of the culture solution, using a control function of the culture apparatus.

A buffer solution that contains no alkali metals such as sodium may be used as the buffer solution for dissolution of the starting substance and pH adjustment according to the invention. Alkali metals are also preferably removed as much as possible from the culture medium components as well, and the main culture medium also preferably contains no alkali metals. This is in order to prevent salt formation between PDC and alkali metals as mentioned above, but the present inventors have found that, for example, when vanillic acid is used as the starting substance, with NaOH for neutral dissolution and pH adjustment, and Na₂HPO₄ has been added to the main culture medium, precipitation begins to be generated in the culture solution when the amount of vanillic acid added to the culture solution exceeds 20 g, with no further accumulation of PDC in the medium thereafter. Such precipitation suggests that the PDC that is produced forms a salt with Na, which is insoluble. When ammonia water was used only for pH adjustment under the same conditions, precipitation that was thought to be insoluble PDC-Na salt was generated when the culture solution supernatant was brought to pH 1 with HCl, during the course of PDC purification once fermentation was complete. Therefore, a "buffer solution that contains no alkali metals", for the purpose of the invention, is a buffer solution containing no alkali metals of a level that will form PDC salts.

The buffer solution that contains no alkali metals to be used for the invention includes, but is not limited to, ammonium salts, trishydroxymethylaminomethane and tricine, preferably ammonium-based buffer solutions, and ammonia water being preferred in particular.

The dissolved oxygen (DO) in the culture solution should maintain a suitable degree of saturation of dissolved oxygen for the microorganism to be cultured. Stirring is preferably controlled for a DO of ≥15% saturation. The dissolved oxygen can be monitored and controlled by functional control provided in the culturing apparatus. It has been found that the dissolved oxygen concentration decreases with consumption of the carbon source such as glucose during the logarithmic growth phase of the microorganism, and increases thereafter with depletion of glucose in the medium.

The temperature of the culture solution is controlled to a temperature that promotes microbial growth and PDC production. The culture solution temperature is controlled to the range of 20 to 40°C and preferably 25 to 30°C, and more preferably it is controlled to 28°C.

The pH adjustment, dissolved oxygen measurement, aeration, stirring speed adjustment and temperature adjustment during culturing may be carried out using a culturing tank having such functions.

It is necessary to add a carbon source, a nitrogen source and inorganic nutrients to the medium or culture solution used for culturing of the microorganism, as nutrients for microorganism growth and/or PDC production.

The term "medium" as used herein refers to both preculture medium and main culture medium. The term "preculture medium" as used herein refers to a medium used for growth of a microorganism on a small scale without addition of starting substances, and the term "main culture medium" refers to a medium to which starting substances are added, also with addition of a preculturing solution that has been used to grow a microorganism in a preculture medium, and wherein PDC is produced by the microorganism.

According to the invention, an amino acid mixture, as an organic nitrogen source, is primarily used as the nitrogen source. The amino acid mixture used for the invention may be yeast extract, malt extract or meat extract, and is preferably yeast extract.

The amino acid mixture may be added to both the preculture medium and the main culture medium. The amino acid mixture is preferably added to the main culture medium in an amount of 3 g/L or more. The amino acid mixture is more preferably added to the main culture medium in an amount of 4 g/L or more, 5 g/L or more or 6 g/L or more, and even more preferably 6.44 g/L or more. Preferably, the amino acid mixture is added to the main culture medium in an amount of 3 to 7 g/L.

A carbon source is also necessary for microbial growth and production of PDC in an adequate amount. The carbon source used for the invention may be glucose, fructose, galactose, xylose, saccharose, maltose or starch, but is preferably glucose.

The carbon source is preferably added to the culture solution by fed-batch addition. As used herein, "fed-batch addition" means a method of continuous addition, e.g. by using a peristaltic pump.

The carbon source is preferably added, when the carbon source that was added during preparation of the main culture medium is depleted during culturing of the microorganism. Microorganisms actively utilize carbon sources and oxygen during the logarithmic growth phase resulting in their growth and proliferation, but depletion of the carbon source lowers oxygen consumption and thus increases dissolved oxygen in the culture solution. Depletion of the carbon source can thus be confirmed based on increased dissolved oxygen. The carbon source is therefore preferably added to the culture solution when the carbon source in the main culture medium is depleted and dissolved oxygen increases, after the start of culturing.

Inorganic nutrients are also necessary to be added for growth of the microorganism. For example, potassium, calcium and magnesium are all necessary as activators for the enzyme reactions in the glycolytic pathway or TCA pathway. Iron carries out electron transport of respiratory chain enzyme cytochromes in aerobic microorganisms. Trace elements such as cobalt, copper and zinc are also necessary.

According to the invention, a divalent metal ion is used as an inorganic nutrient. Divalent metal ions to be used for the invention include metal ions of magnesium, calcium, iron (II), copper(II), zinc, barium, cobalt, nickel, manganese, chromium(II) and boron. The divalent metal ion may be added in the form of a salt, with salts of divalent metal ions including inorganic acid salts such as hydrochlorides, nitrates, sulfates and phosphates of metal ions, and organic acid salts of acetic acid, oxalic acid, citric acid, tartaric acid, fumaric acid, maleic acid, malic acid, cyanic acid and thiocyanic acid. Preferred divalent metal ions to be added are magnesium sulfate heptahydrate, iron sulfate heptahydrate, magnesium oxide, calcium carbonate, zinc sulfate heptahydrate, manganese sulfate tetrahydrate, copper sulfate pentahydrate, cobalt sulfate heptahydrate and boric acid.

The divalent metal ion may be added to both the preculture medium and the main culture medium. To 1 L of main culture medium it is preferred to add 750 mg of MgSO₄·7H₂O, 71.25 mg of FeSO₄·7H₂O, 80.625 mg of MgO, 15 mg of CaCO₃, 10.8 mg of ZnSO₄·7H₂O, 8.4 mg of MnSO₄·4H₂O, 1.875 mg of CuSO₄·5H₂O, 2.1 mg of CoSO₄·7H₂O and 0.45 mg of H₃BO₃.

The amounts and methods of addition of the aforementioned nutrients to the medium, and the timing of their addition, may be set based on the results of metabolome analysis.

The microorganism to be used for the invention is not particularly restricted so long as it is a PDC-producing microorganism, but it is preferably a transformant of *Pseudomonas* (*Pseudomonas*) bacterium that has been transfected with a recombinant vector, and more preferably a transformant of *Pseudomonas putida* that has been transfected with a recombinant vector.

The vector to be used for the invention is preferably pDVZ21X or pKTVPVABC. A method for constructing these vectors is disclosed in Japanese Unexamined Patent Publication No. 2011-067139, the aforementioned vector pKTVPVABC being identical to vector pVaPoLigVABC mentioned in this publication.

The *Pseudomonas putida* used for the invention may also be *Pseudomonas putida* strain PpY1100.

The starting substances to be used in the method of the invention include vanillin, vanillic acid, syringaldehyde, syringic acid, p-hydroxybenzoic acid, protocatechuic acid and *p-*hydroxybenzaldehyde, which are derived from plant components or petroleum components, or chemically synthesized. Preferably, the starting substances are vanillic acid or vanillin, or aromatic mixtures containing vanillin and/or vanillic acid, with vanillic acid or vanillin being especially preferred.

The starting substances are dissolved in a buffer solution that contains essentially no alkali metals. As mentioned above, "contains no alkali metals", means that the buffer solution contains no alkali metals of a level that will form PDC salts. The starting substances are preferably added to the culture solution by fed-batch addition.

The method of the invention can produce PDC at high yield. For example, PDC is produced by the method of the invention in an amount of 30 g or more, 50 g or more, 60 g or more, 70 g or more, 80 g or more, 90 g or more, 110 g or more or 120 g or more, per culture solution (L). Preferably, PDC is produced in an amount of 40 g or more, 80 g or more or 100 g or more per culture solution (L). The range of PDC production by the method of the invention may be 30 to 130 g, 40 to 130 g, 30 to 50 g, 70 to 130 g or 80 to 130 g, per culture solution (L). While a high yield of PDC is preferred in the method of the invention, it is sufficient if production is in a high amount so long as growth of the microorganism is not adversely affected.

The microorganism culturing time is not particularly restricted, but it may be a time necessary for adequate growth of the microorganism and production of PDC, such as culturing for 30 hours or longer, 40 hours or longer, 60 hours or longer, 80 hours or longer or 100 hours or longer, for example. The culturing time is preferably 60 to 100 hours.

Using a buffer solution that contains no alkali metals during culturing is also advantageous from the viewpoint of purification of the PDC that is produced. After culturing is complete, the reaction is suspended using hydrochloric acid to lower the pH of the culture solution to pH 1 or below, during which time an insoluble salt (sodium salt) will form in the culture solution if a buffer solution containing an alkali metal such as sodium hydroxide was used, and the culture solution therefore becomes opaque. Once such formation occurs it is no longer possible to separate and purify the PDC without using strong cation exchange chromatography. In the method of the present application which uses a buffer solution that contains no alkali metals, however, salt formation does not occur even after hydrochloric acid treatment, allowing the PDC to be separated by convenient means such as ethyl acetate extraction.

### EXAMPLES

The present invention will now be described in greater detail by examples, with the understanding that the invention is not limited to these examples.

The bacterial strains *P. putida* (pDVZ21X/PpY1100) and *P. putida* (pKTVPVABC/PpY1100) used herein are *Pseudomonas putida* PpY1100 transformants containing vector pDVZ21X and pKTVPVABC, respectively, the vectors and transformants being prepared according to the method described in Japanese Unexamined Patent Publication No. 2011-067139.

### Example 1: PDC production from vanillic acid (1)

### 1-1 Bacterial strain

*P. putida* (pDVZ21X/PpY1100) was flask cultured overnight in LB medium (10 ml) containing 50 µg/ml kanamycin, and then mixed with an equivalent amount of a 60% glycerol solution. The mixture was dispensed into 1.5 ml aliquots and stored at -80°C.

This was used as a working cell bank (WCB) for the main experiment.

### 1-2 Medium starting materials and reagents

The medium starting materials and reagents used were the following.
Ammonium sulfate (Wako)
Potassium dihydrogen phosphate (Wako)
Disodium hydrogen phosphate 12-hydrate (Wako)
Diammonium hydrogen phosphate (Wako)
Bacto Yeast Extract (B&D)
Magnesium sulfate (Wako)
Magnesium oxide (Wako)
Calcium carbonate (Wako)
Iron sulfate heptahydrate (Wako)
Zinc sulfate heptahydrate (Wako)
Manganese sulfate tetrahydrate (Alfa Aesar)
Copper sulfate pentahydrate (Wako)
Cobalt sulfate heptahydrate (Wako)
Boric acid (Wako)
Hydrochloric acid (Nacalai)
Kanamycin sulfate (Wako)
Vanillic acid (Taizhou Zhongda Chemical, China)
Antifoaming agent: ADEKANOL LG-295S (Adeka Corp.)
Ammonia water (Wako)

### 1-3 Culturing conditions (2 L culturing tank)

### 1-3-1 Devices

The following devices were used for the culturing.
2 L culturing tank: MDL-2 Marubishi Bioengineering Co., Ltd.
Shake culture apparatus: BR-30LF Tietech Co., Ltd. (for preculturing; 500 mL baffle-equipped flask)

### 1-3-2 Preculturing

### (1) Preparation of preculture medium

The following solution and medium were prepared first.
[1] SSS solution composition: 100 mL

| | |
|---|---|
| MgO | 1.075 g |
| CaCO₃ | 0.20 g |
| FeSO₄·7H₂O | 0.45 g |
| ZnSO₄·7H₂O | 0.144 g |
| MnSO₄·4H₂O | 0.112 g |
| CuSO₄·5H₂O | 0.025 g |
| CoSO₄·7H₂O | 0.028 g |
| H₃BO₃ | 0.006 g |
| 12N HCl | 5.136 ml |

[2] W. stock sol. II composition: 1 L

| | |
|---|---|
| MgSO₄·7H₂O | 10 g |
| FeSO₄·7H₂O | 0.5 g |

The components were dissolved in 900 mL of deionized water, 100 mL of the SSS solution of [1] was added, and the mixture was subjected to mechanical sterilization with a 0.2 mm filter.
[3] Preculturing basal medium

| | |
|---|---|
| (NH₄)₂SO₄ | 5.6 g |
| KH₂PO₄ | 3.6 g |
| Na₂HPO₄·12H₂O | 20.6 g |
| Bacto Yeast Extract | 4.2 g |
| Tap water | 890 mL |

The solution and medium were used to prepare preculture medium in the following manner.

### <Preculture medium (W-modified medium) 1 L>

After placing 89 mL of the preculturing basal medium of [3] in a 500 ml baffle-equipped flask, the mixture was autoclaved. The following three different solutions were added after cooling.
- 10 mL of glucose solution, 18 g/100 mL (sterilized for 20 minutes at 121°C).
- 0.25 mL of kanamycin sulfate solution, 10 mg/mL (filtered with a 0.2 µm filter).
- 1 mL of W. stock sol. II of [2].

### (2) Preculturing conditions

A 1 ml portion of WCB glycerol stock was transferred into a baffle-equipped flask containing the medium for preculturing. The mixture was then shake cultured for 8 to 11 hours at 28°C, 150 to 160 rpm/min to prepare a preculturing solution.

### 1-3-3 Main culturing

### (1) Preparation of main culture medium

The following solution and medium were prepared first.
[1] 50% glucose solution
After dissolving 150 g of glucose in 150 g of water, the solution was sterilized at 121°C for 20 minutes.
[2] W. stock sol. II (for main culturing) composition: 1 L

| | |
|---|---|
| MgSO₄·7H₂O | 25 g |
| FeSO₄·7H₂O | 1.25 g |
| SSS solution (as described in 1-3-2 [1]) | 250 ml |

[3] Main culturing basal medium

| | |
|---|---|
| (NH₄)₂SO₄ | 6.70 g |
| KH₂PO₄ | 4.31 g |
| (NH₄)₂HPO₄ | 9.10 g |
| Bacto Yeast Extract | 3.22 g |
| Dissolved in tap water | (0.95 L) |

[4] Vanillic acid feed solution

After suspending 100 g of vanillic acid in 443 g of water, it was dissolved (pH 7 to 7.5) while adding 28% ammonia water dropwise (40 to 45 mL of 28% ammonia water). It was then sterilized with a 0.2 µm filter.

The solution and medium were used to prepare main culture medium in the following manner.

### <Main culture medium (W-modified medium) 1 L>

After transferring the main culturing basal medium of [3] into a culturing tank, 0.22 g of ADEKANOL LG295S antifoaming agent was added. The mixture was sterilized at 121°C for 20 minutes, and the following two different solutions were added after cooling.
- 68 g of glucose solution of [1]
- 15 mL of W. stock sol. II of [2] (for main culturing) (filtered with 0.2 µm filter)

### (2) Main culturing conditions

1) After placing 0.4 L of main culture medium in a 2 L culturing tank, 0.05% of the preculturing solution was added (OD 660 nm = 5).
2) With stirring control for dissolved oxygen (DO) ≥15% sat. at 28°C, 200 to 800 rpm, culturing was carried out at an aeration rate of 1 v.v.m.
3) 14% ammonia water was automatically added to the culture solution to maintain pH 6.5 or higher.
4) After the glucose added to the main culture medium was consumed and the dissolved oxygen increased (approximately 2 hours from the start of culturing), fed-batch addition of a 50% glucose solution to the culture solution was initiated at 0.08 g/min.
5) During the same period, fed-batch addition of the vanillic acid solution of [4] was initiated at 0.11 to 0.17 g/min.
6) Antifoaming agent was intermittently added upon the occurrence of foaming (at 20 to 40 hours of culturing).
7) The procedure was carried out with periodic sampling and bacterial concentration measurement (OD 660 nm).
8) Upon completion of the reaction, HCl was added to the culture solution for acidification (~pH 1) from which the bacteria had been centrifugally removed, and after ethyl acetate extraction, vanillic acid conversion was confirmed by TLC.
9) The culture solution was further diluted with methanol (1/10 to 1/200), and the amount of PDC produced in the supernatant was measured by HPLC.

### 1-4 Results

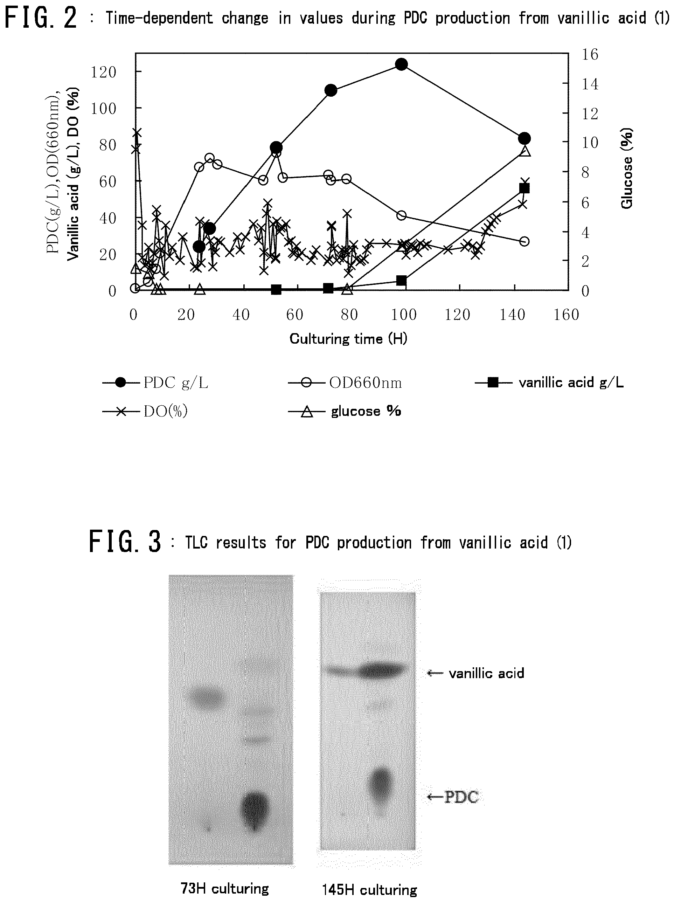

The PDC concentration at 73 hours after the start of culturing was 110 g/L. Accumulation of vanillic acid and glucose began at about 80 hours of culturing, with the vanillic acid feed reaching 140 g at 99 hours of culturing, and the PDC production increasing to 123 g/L, after which the PDC concentration fell and the reaction essentially stopped. Fig. 2 shows time-dependent change in each value measured during culturing, and Fig. 3 shows the results of TLC at 73 hours and 145 hours of culturing.

### Example 2: PDC production from vanillic acid (2)

PDC was produced by the same method as in I above, except that the following were changed in the "1-3-3 Main culturing":
- The amount of Bacto Yeast Extract added to the main culturing basal medium was increased from 3.22 g to 6.44 g;
- The amount of W. stock sol. II (for main culturing) added to the main culture medium was increased from 15 mL to 30 mL;
- The amount of main culture medium placed in the 2 L culturing tank in "(2) Main culturing conditions" (step 1) was changed from 0.4 L to 0.5 L.

### II-4 Results

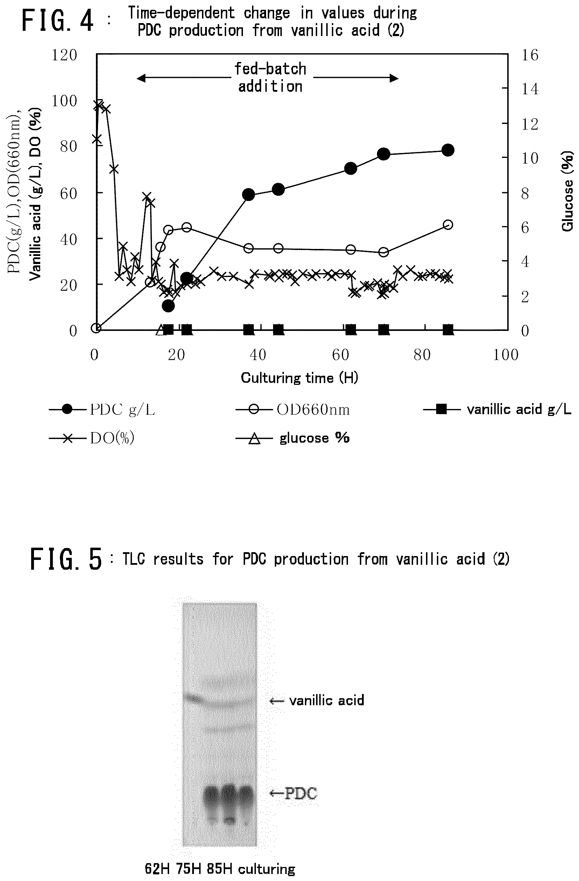

At 85 hours of culturing, 100 g of vanillic acid was completely converted and the amount of PDC was 80 g/L. Fig. 4 shows time-dependent change in each value measured during culturing, and Fig. 5 shows the results of TLC.

### Example 3: PDC production from vanillin

III-1 Bacterial strain
   *P. putida* (pKTVPVABC/PpY1100) was flask cultured overnight in LB medium (10 ml) containing 50 µg/ml kanamycin, and then mixed with an equivalent amount of a 60% glycerol solution. The mixture was dispensed into 1.5 ml aliquots and stored at -80°C. This was used as the WCB for the main experiment.
III-2
   - Medium starting materials and reagents
      Vanillin (Wako)
      * Otherwise same as I above.
III-3 Culturing conditions (2 L culturing tank)
III-3-1 Device
   Same as I above.
III-3-2 Preculturing
   Same as I above.
III-3-3 Main culturing

### (1) Preparation of main culture medium

The following solution and medium were prepared first.
[1] 50% glucose solution
After dissolving 150 g of glucose in 150 g of water, the solution was sterilized at 121°C for 2 minutes.
[2] W. stock sol. II (for main culturing) composition: 1 L

| | |
|---|---|
| MgSO₄·7H₂O | 25 g |
| FeSO₄·7H₂O | 1.25 g |
| SSS solution (as described in 1-3-2 [1]) | 250 ml |

[3] Main culturing basal medium

| | |
|---|---|
| (NH₄)₂SO₄ | 6.70 g |
| KH₂PO₄ | 4.31 g |
| (NH₄)₂HPO₄ | 9.10 g |
| Bacto Yeast Extract | 3.22 g |
| Dissolved in tap water | (0.95 L) |

[4] Vanillin feed solution
After suspending 50 g of vanillin in 500 g of water, it was dissolved (pH 7.5 to 8.2) while adding 28% ammonia water dropwise (20 to 25 mL of 28% ammonia water). It was then sterilized with a 0.2 µm filter.
[5] Added yeast extract solution
A 13 g portion of yeast extract was dissolved in 94 g of water and the mixture was sterilized at 121°C for 20 minutes.

The solution and medium were used to prepare main culture medium in the following manner.

### <Main culture medium (W-modified medium) 1 L>

After transferring the main culturing basal medium of [3] into a culturing tank, 0.22 g of ADEKANOL LG295S antifoaming agent was added. The mixture was sterilized at 121°C for 20 minutes, and the following two different solutions were added after cooling.
- 68 g of 50% glucose solution of [1]
- 15 mL of W. stock sol. II of [2] (filtered with 0.2 µm filter)

### (2) Main culturing conditions

1) After placing 0.6 L of main culture medium in a 2 L culturing tank, 2% of the preculturing solution was added (OD 660 nm = 10).
2) With stirring control for dissolved oxygen (DO) ≥15% sat., culturing was carried out at an aeration rate of 1 v.v.m, at 28°C, 200 to 800 rpm.
3) 14% ammonia water was automatically added to the culture solution for control to pH 6.5.
4) After the glucose added to the main culture medium was consumed (DO increased), fed-batch addition of glucose solution to the culture solution was initiated (0.10 g/min).
5) During the same period, fed-batch addition of the vanillin solution of [4] was initiated (0.12 to 0.14 g/L/min).
6) A mixture of 10 mL of the yeast extract solution of [5], and 6 mL of W. stock sol. II (for main culturing) was added at 31 hours and 81 hours.
7) Antifoaming agent was intermittently added upon the occurrence of foaming (at 20 to 70 hours of culturing).
8) The procedure was carried out with periodic sampling and bacterial concentration measurement (OD 660 nm).
9) Upon completion of the reaction, the culture solution was brought to acidic pH (about pH 1), ethyl acetate extraction was performed and vanillin conversion was confirmed by TLC.
10) The culture solution was further diluted with methanol (1/10 to 200), and the amount of PDC produced in the supernatant was measured by HPLC.

### III-4 Results

The amount of PDC production was 40 g/L at 80 hours of culturing.

## Claims

1. A method of producing 2-pyrone-4,6-dicarboxylic acid (PDC) by culturing a microorganism that produces PDC, wherein the method comprises:
dissolving the starting substance for production of PDC in a buffer solution that contains no alkali metals; and
adjusting the pH of a culture solution with a buffer solution that contains no alkali metals.

2. The method according to claim 1, wherein:
the dissolution of the starting substance for production of PDC in the buffer solution that contains no alkali metals comprises culturing the microorganism in the culture solution containing the buffer solution that contains no alkali metals, in which the starting substance has been dissolved; and
the pH adjustment of the culture solution with the buffer solution that contains no alkali metals comprises appropriately adjusting the pH of the culture solution with the buffer solution that contains no alkali metals when the pH has been decreased during culturing.

3. The method according to claim 1 or 2, wherein the buffer solution that contains no alkali metals is an ammonium-based buffer solution.

4. The method according to claim 3, wherein the ammonium-based buffer solution is ammonia water.

5. The method according to any one of claims 1 to 4, which further comprises adding an amino acid mixture to the culture solution.

6. The method according to claim 5, wherein yeast extract is used as the amino acid mixture.

7. The method according to claim 6, wherein the yeast extract is added to the culture solution in an amount of 3 g/L or more.

8. The method according to any one of claims 1 to 7, which further comprises adding a carbon source to the culture solution.

9. The method according to claim 8, wherein the carbon source is glucose.

10. The method according to claim 8 or 9, wherein the carbon source is added by fed-batch addition.

11. The method according to any one of claims 1 to 10, which further comprises adding a divalent metal ion to the culture solution.

12. The method according to claim 11, wherein the divalent metal ion is composed of MgSO₄·7H₂O, FeSO₄·7H₂O, MgO, CaCO₃, ZnSO₄·7H₂O, MnSO₄·4H₂O, CuSO₄·5H₂O, CoSO₄·7H₂O and H₃BO₃.

13. The method according to any one of claims 1 to 12, which comprises adding the starting substance to the culture solution by fed-batch addition.

14. The method according to any one of claims 1 to 13, wherein the microorganism is a transformant of a *Pseudomonas* bacterium that has been transfected with a recombinant vector.

15. The method according to claim 14, wherein the *Pseudomonas* bacterium is *Pseudomonas putida.*

16. The method according to any one of claims 1 to 15, wherein the starting substance is vanillic acid or vanillin, or a mixture of aromatic substances comprising vanillin and/or vanillic acid.

17. The method according to any one of claims 1 to 16, wherein PDC is produced at 40 g or more per culture solution (L).

18. The method according to claim 17, wherein PDC is produced at 80 g or more per culture solution (L).

19. The method according to claim 18, wherein PDC is produced at 100 g or more per culture solution (L).
